# EUROPEAN PATENT APPLICATION

(11) **EP 4 442 271 A1**
(43) Date of publication of application: **09.10.2024**
(21) Application number: 22900559.0
(22) Date of filing: 30.11.2022
(51) Int. Cl.: A61K 39/05, A61K 39/385, A61K 39/39, A61K 47/26, A61K 47/64, A61K 9/08, A61P 35/00, C07K 14/34, C07K 14/485

(54) **RECOMBINANT HEGF-CRM197 TUMOR THERAPEUTIC VACCINE FORMULATION**

(30) Priority: 01.12.2021 CN 202111448592
(71) Applicant: SHANGHAI HUIMMUTECH BIOTECHNOLOGY CO., LTD, Shanghai 200120 (CN)
(72) Inventor: ZHANG, Wenyao, Shanghai 200120 (CN); CHEN, Guoyou, Shanghai 200120 (CN)
(74) Representative: Petraz, Gilberto Luigi
(86) International application number: PCT/CN2022/135608
(87) International publication number: WO 2023/098755

(57) **Abstract**

Provided in the present invention is a recombinant hEGF-CRM197 tumor therapeutic vaccine formulation. Specifically, the formulation of the present invention contains a therapeutically effective amount of a conjugate of a recombinant human epidermal growth factor (hEGF) and a diphtheria toxin mutant (CRM197), a phosphate base buffer solution with the pH in a range of 7.5-8.5, a polysorbate 20 surfactant and optionally a monosaccharide or disaccharide. The protein conjugate molecule in the formulation of the present invention can break immune tolerance and induce the production of an anti-human epidermal growth factor antibody in the human body; In addition, the protein conjugate molecule produces a lower proportion of polymers, and has a more uniform molecular weight distribution in the buffer, and better stability. Therefore, the formulation of the present invention can achieve large-scale production and can be stably stored for a long time.

## Description

### TECHNICAL FIELD

The present invention relates to the field of biomedicine, in particular to a preparation method and use of a recombinant hEGF-CRM197 tumor therapeutic vaccine formulation.

### BACKGROUND

In recent years, with the rapid development and cross penetration of related disciplines such as oncology, immunology, and molecular biology, the use of tumor vaccines that activate the body's specific anti-tumor immune function to treat cancer has become a research hotspot in the field of malignant tumor treatment. Tumor vaccines belong to active immunotherapies which activate tumor specific immune responses through tumor associated antigens to achieve the goal of killing and clearing tumor cells, wherein protein coupled vaccines or polypeptide coupled vaccines based on carrier proteins are one of the hotspots in the development of therapeutic vaccines.

Up to now, research on the development of protein chemically coupled vaccine formulations is relatively rare. Protein conjugates based on chemical coupling often have characteristics such as complex molecular forms, complex structures, and strong hydrophobicity, which pose new challenges for the development of therapeutic chemically coupled drug formulations.

### SUMMARY OF THE INVENTION

The purpose of the present invention is to provide a stable recombinant hEGF-CRM197 tumor therapeutic vaccine formulation, which comprises a conjugate of recombinant human epidermal growth factor (hEGF) and diphtheria toxin mutant, a phosphate buffer with a pH range of 7.0-8.5, and a surfactant polysorbate 20, and/or an optional monosaccharide or disaccharide, as well as a method for preparing the formulation.

In the first aspect of the present invention, it provides a recombinant hEGF-CRM197 tumor therapeutic vaccine formulation, which comprises:
(a) a recombinant human epidermal growth factor (hEGF)-diphtheria toxin mutant CRM197 conjugate;
(b) a phosphate basic buffer with a pH range of 7.0-8.5, wherein the conductivity of the buffer is not higher than 10mS/cm; and
(c) a protein stabilizer;
wherein, the protein stabilizer comprises: surfactant polysorbate 20 and an optional monosaccharide or disaccharide.

In another preferred embodiment, the conjugate is obtained by coupling a recombinant human epidermal growth factor (hEGF) and a diphtheria toxin mutant CRM197 with a chemical crosslinking agent glutaraldehyde.

In another preferred embodiment, the conductivity of the buffer is not higher than 3mS/cm.

In another preferred embodiment, the conjugate contains a recombinant human epidermal growth factor that is covalently crosslinked with a diphtheria toxin mutant through glutaraldehyde.

In another preferred embodiment, the conjugate has a structure as shown in Formula (I):

hEGF-L-CRM197 (I)

wherein,
hEGF is the recombinant human epidermal growth factor (hEGF);
CRM197 is the diphtheria toxin mutant CRM197;
L is a linker connecting the recombinant human epidermal growth factor with the diphtheria toxin mutant CRM197, which is a covalent bond formed by glutaraldehyde crosslinking; and
"-" is a chemical bond.

In another preferred embodiment, the crosslinking molar ratio of the recombinant human epidermal growth factor (hEGF)/diphtheria toxin mutant CRM197 in the conjugate is 6:1.

In another preferred embodiment, the recombinant human epidermal growth factor (hEGF) is an EGF molecule or a mutant thereof containing human epidermal growth factor (hEGF) biological activity, and the EGF biological activity refers to the ability of the EGF molecule to stimulate or induce signal transduction in cells containing EGFR receptors.

In another preferred embodiment, the recombinant human epidermal growth factor (hEGF) is a full-length sequence or a partial fragment thereof of a human epidermal growth factor (hEGF).

In another preferred embodiment, the full-length sequence of the human epidermal growth factor (hEGF) has an amino acid sequence as shown in SEQ ID NO: 1.

In another preferred embodiment, the recombinant human epidermal growth factor (hEGF) is obtained by truncating 2-3 amino acids from the N-terminus or C-terminus of the full-length sequence of the human epidermal growth factor (hEGF).

In another preferred embodiment, the recombinant human epidermal growth factor (hEGF) has an amino acid sequence as shown in SEQ ID NO: 3.

In another preferred embodiment, the diphtheria toxin mutant CRM197 has an amino acid sequence as shown in SEQ ID NO: 2.

In another preferred embodiment, the basic buffer is a phosphate buffer with a phosphate concentration of 1-40 mmol/L.

In another preferred embodiment, the phosphate buffer comprises sodium phosphate buffer (NaH₂PO₄&Na₂HPO₄) and potassium phosphate buffer (K₂HPO₄&KH₂PO₄).

In another preferred embodiment, the basic buffer is a 20 mmol/L phosphate buffer with a pH of 8.0.

In another preferred embodiment, the basic buffer is a 10 mmol/L phosphate buffer with a pH of 7.5.

In another preferred embodiment, the basic buffer is a 5 mmol/L phosphate buffer with a pH of 7.0.

In another preferred embodiment, the concentration of polysorbate 20 in the formulation is 0.001% to 0.1% (v/v).

In another preferred embodiment, the concentration of polysorbate 20 in the formulation is 0.005% to 0.02% (v/v).

In another preferred embodiment, the concentration of polysorbate 20 in the formulation is 0.1% (v/v).

In another preferred embodiment, the concentration of polysorbate 20 in the formulation is 0.02% (v/v).

In another preferred embodiment, the concentration of polysorbate 20 in the formulation is 0.01% (v/v).

In another preferred embodiment, the concentration of polysorbate 20 in the formulation is 0.005% (v/v).

In another preferred embodiment, the concentration of polysorbate 20 in the formulation is 0.001% (v/v).

In another preferred embodiment, the optional monosaccharide or disaccharide is selected from the group consisting of: sucrose, glucose, mannitol, maltose, and a combination thereof.

In another preferred embodiment, the optional monosaccharide or disaccharide is sucrose, and the concentration of sucrose in the formulation is 0-4% (mass/volume).

In another preferred embodiment, the concentration of sucrose in the formulation is 2% (m/v).

In another preferred embodiment, the concentration of sucrose in the formulation is 4% (m/v).

In another preferred embodiment, the optional monosaccharide or disaccharide is maltose, and the concentration of maltose in the formulation is 2% (m/v).

In another preferred embodiment, the formulation is a liquid formulation.

In another preferred embodiment, the formulation is an injection.

In another preferred embodiment, the content of the conjugate of recombinant human epidermal growth factor (hEGF) and diphtheria toxin mutant in the formulation is 0.4-3.2 mg/ml.

In another preferred embodiment, the formulation does not contain free amino acids.

In another preferred embodiment, the formulation has the following characteristics: the formulation remains stable for at least 3 months, preferably 6 months, under a temperature condition of 37 °C.

In another preferred embodiment, the formulation remains stable for 6 months under a temperature condition of 25 °C ± 2 °C or 37 °C ± 2 °C.

In another preferred embodiment, "remain stable" refers to the absence of significant changes in key quality parameters such as appearance, visible foreign objects, protein content, purity, molecular weight distribution, *in vitro* relative potency, and *in vivo* biological activity of the formulation.

In the second aspect of the present invention, it provides a method for preparing the recombinant hEGF-CRM197 tumor therapeutic vaccine formulation of the first aspect of the present invention, which comprises the following steps:
(i) providing a purified recombinant human epidermal growth factor (hEGF) protein and a diphtheria toxin mutant CRM197 protein;
(ii) reacting the hEGF protein and CRM197 protein with a chemical crosslinking agent glutaraldehyde, thereby obtaining the hEGF-CRM197 conjugate;
(iii) purifying the hEGF-CRM197 conjugate, thereby obtaining the purified hEGF-CRM197 conjugate;
(iv) exchanging the purified hEGF-CRM197 conjugate into a basic buffer supplemented with a protein stabilizer, thereby obtaining the recombinant hEGF-CRM197 tumor therapeutic vaccine formulation.

In another preferred embodiment, the amino acid sequence of the recombinant human epidermal growth factor (hEGF) protein is shown in SEQ ID NO: 3.

In another preferred embodiment, the amino acid sequence of the diphtheria toxin mutant CRM197 protein is shown in SEQ ID NO: 2.

In another preferred embodiment, the reaction condition in step (ii) is room temperature (25 °C ± 2 °C) and the reaction time is 2 to 3 hours.

In another preferred embodiment, the crosslinking agent is glutaraldehyde.

In another preferred embodiment, ultrafiltration is used for purification in step (iii).

In another preferred embodiment, ultrafiltration is used for medium exchange in step (iv).

In another preferred embodiment, the basic buffer is a phosphate buffer with a pH range of 7.0-8.5, and a phosphate concentration of 1-40 mmol/L.

In another preferred embodiment, the conductivity of the basic buffer is not higher than 3mS/cm.

In another preferred embodiment, the protein stabilizer comprises a surfactant polysorbate 20 and an optional monosaccharide or disaccharide.

In another preferred embodiment, the concentration of polysorbate 20 is 0.005% to 0.02% (v/v).

In another preferred embodiment, the optional monosaccharide or disaccharide is selected from the group consisting of: sucrose, glucose, mannitol, maltose, and a combination thereof.

In the third aspect of the present invention, it provides a composition, which comprises (a) the tumor therapeutic vaccine formulation of the first aspect of the present invention; and (b) a pharmaceutically acceptable carrier.

In another preferred embodiment, the composition is a pharmaceutical composition or a vaccine composition.

In another preferred embodiment, the vaccine composition further comprises an adjuvant.

In another preferred embodiment, the adjuvant comprises a particulate adjuvant and a non-particulate adjuvant.

In another preferred embodiment, the particulate adjuvant is selected from the group consisting of: aluminum salts, water-in-oil emulsions, oil-in-water emulsions, nanoparticles, micro-particles, liposomes, immunostimulatory complexes, and a combination thereof.

In another preferred embodiment, the non-particulate adjuvant is selected from the group consisting of: muramyl dipeptides and derivatives thereof, saponins, lipid A, cytokines, derived polysaccharides, bacterial toxins, microorganisms and products thereof such as Mycobacterium (Mycobacterium tuberculosis, Bacillus Calmette Guerin), Curtobacterium, Bordetella pertussis, propolis, and a combination thereof.

In another preferred embodiment, the adjuvant is selected from the group consisting of: Montanide ISA 51 VG, aluminium phosphate, MF59, AS04, and a combination thereof.

In another preferred embodiment, the composition is prepared as a liquid formulation.

In another preferred embodiment, the composition is prepared as an injection, suspension, or spray.

In the fourth aspect of the present invention, it provides a use of the tumor therapeutic vaccine formulation of the first aspect of the present invention in the manufacture of a medicament for treating and/or preventing a disease.

In another preferred embodiment, the disease is a cancer or tumor.

In another preferred embodiment, the tumor (or cancer) comprises a solid tumor.

In another preferred embodiment, the solid tumor is selected from the group consisting of: lung cancer, non-small cell lung cancer, colorectal cancer, breast cancer, liver cancer, gastric cancer, esophageal cancer, pancreatic cancer, melanoma, kidney cancer, prostate cancer, cervical cancer, ovarian cancer, nasopharyngeal cancer, oral cancer, osteosarcoma, cerebral glioma, bladder cancer, and a combination thereof.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

Figure 1 shows the antibody titer detection of mice immunized with the recombinant hEGF-CRM197 conjugate.
Figure 2 shows the molecular weight distribution (SEC-HPLC) profile of the polysorbate 80-containing formulation (No. a7) after accelerated stability testing at 37 °C for 1 month.
Figure 3 shows the molecular weight distribution (SEC-HPLC) profile of the polysorbate 20-containing formulation (No. a13) after accelerated stability testing at 37 °C for 1 month.
Figure 4 shows the comparison of the molecular weight distribution (SEC-HPLC) profiles of the recombinant hEGF-CRM197 tumor therapeutic vaccine formulation (0.01% Tween20, 2% sucrose, 20mM PB, pH 8.0, 1.6mg/ml) in initial inspection and after accelerated stability testing at 25 °C for 6 months.
Figure 5 shows the comparison of the biological activities of the recombinant hEGF-CRM197 tumor therapeutic vaccine formulation (0.01% Tween20, 2% sucrose, 20mM PB, pH 8.0, 1.6mg/ml) in initial inspection and after accelerated stability testing at 25 °C for 6 months.
Figure 6 shows the antibody titer detection of antibodies induced by the recombinant hEGF-CRM197 tumor therapeutic vaccine formulation in cynomolgus monkeys.
Figure 7 shows the effect of the recombinant hEGF-CRM197 tumor therapeutic vaccine formulation on EGF content in the serum of cynomolgus monkeys.
Figure 8 shows the antibody titer detection results of mice immunized with rhEGF-P64k conjugate (CIMAvax-EGF) and the recombinant hEGF-CRM197 conjugate.
Figure 9 shows the detection results of rhEGF-P64k conjugate (CIMAvax-EGF) and the recombinant hEGF-CRM197 conjugate blocking the binding of EGF and EGFR *in vitro.*

### DETAILED DESCRIPTION

After extensive and in-depth research, the inventors unexpectedly developed a stable and storable recombinant hEGF-CRM197 tumor therapeutic vaccine formulation for the first time. The vaccine formulation of the present invention comprises a conjugate of recombinant human epidermal growth factor (hEGF) and diphtheria toxin mutant, a phosphate buffer with a pH range of 7.0-8.5, a surfactant polysorbate 20 (Tween 20), and/or an optional monosaccharide or disaccharide. Experimental results have shown that the vaccine formulation of the present invention has a low proportion of polymers and high molecular weight uniformity. After accelerated treatment at 25 °C ± 2 °C and 37 °C ± 2 °C for 6 months, there were no changes in its quality attributes such as appearance, visible foreign objects, molecular weight distribution, protein content, EGF antigen content, and biological activity. Therefore, the recombinant hEGF-CRM197 tumor therapeutic vaccine formulation of the present invention can be used as an efficient drug for tumor treatment and/or prevention, which can be rapidly mass-produced and stored for a long time. On this basis, the present invention has been completed.

### Term

In order to make this disclosure easier to understand, certain technical and scientific terms are specifically defined below. Unless specifically defined otherwise herein, all other technical and scientific terms used herein have the meanings commonly understood by those skilled in the art to which the present invention belongs.

As used herein, when used in reference to a specific enumerated value, the term "about" means that the value may vary by no more than 1% from the enumerated value. For example, as used herein, the expression "about 100" includes all values between 99 and 101 (e.g., 99.1, 99.2, 99.3, 99.4, etc.).

As used herein, the term "treatment" refers to the administration of a therapeutic agent comprising the tumor-targeting vaccine and a composition thereof of the present invention to a patient, either internally or externally, wherein the patient has one or more disease symptoms on which the therapeutic agent is known to have a therapeutic effect. Generally, the therapeutic agent is administered to the patient in an amount that is effective to alleviate one or more disease symptoms (therapeutically effective amount).

As used herein, the term "optional" or "optionally" means that the subsequently described event or circumstance may occur but not necessarily occur. For example, "optional monosaccharide or disaccharide" refers that the specific monosaccharide or disaccharide may be present, but is not necessarily required, and there may be 1, 2 or 3 of it.

### Epidermal Growth Factor (EGF)

Epidermal growth factor (EGF) is a heat-resistant single chain low molecular weight peptide composed of 53 amino acid residues. After EGF specifically recognizes and binds to EGF receptors on target cells, a series of biochemical reactions occur, ultimately promoting DNA synthesis and mitosis in target cells. EGF has no glycosylated sites, and it is very stable, as well as heat-resistant and acid-resistant. It is widely present in body fluids and various glands, mainly synthesized by the submandibular gland and duodenum. It has been found in the vast majority of body fluids in the human body, with a specific increase in content in milk, urine, and semen, but a lower concentration in serum. EGF has a wide range of effects and is of great significance in estimating tumor prognosis and selecting treatment options.

The epidermal growth factor (EGF) of the present invention is human epidermal growth factor (hEGF), specifically a recombinant human epidermal growth factor (hEGF), which is obtained by truncating 2-3 amino acids at the N-terminus or C-terminus of the full-length sequence of human epidermal growth factor (hEGF) (as shown in SEQ ID NO: 1).

In a preferred embodiment of the present invention, the recombinant human epidermal growth factor of the present invention is obtained by truncating two amino acids from the C-terminus of the full-length sequence of human epidermal growth factor (hEGF), which has an amino acid sequence shown in SEQ ID NO: 3:

NSDSECPLSHDGYCLHDGVCMYIEALDKYACNCVVGYIGERCQY
RDLKWWE (SEQ ID NO: 3).

### Diphtheria toxin mutant CRM197

Diphtheria toxin (DT) is an exotoxin produced by Corynebacterium diphtheriae, which is infected with beta phages. It is present in the components of the DPT vaccine used in clinical practice. Its safety has been verified through years of clinical use, with rare serious adverse reactions. Currently, there have been no reports of allergic reactions caused by diphtheria components.

The molecule of diphtheria toxin consists of 535 amino acid residues, which spatially consist of a relatively independent catalytic domain (1-193AAs), a transmembrane domain (205-378AAs), and a receptor-binding domain (386-535AAs). The transmembrane domain and receptor-binding domain themselves are non-toxic, and their function is to transduce the catalytic domain into the cell through the binding to cell surface receptors.

CRM197 is a diphtheria toxin mutant obtained from a strain of diphtheria virulence gene mutant strain, with a length of 536 amino acids. Specifically, the glycine at position 52 of the diphtheria toxin is mutated to a glutamic acid, and the mutant has an amino acid sequence as shown in SEQ ID NO: 2. The mutant toxin A fragment cannot bind to the intranuclear elongation factor II, causing it to lose its cytotoxic effect, but its antigenicity and immunogenicity remain largely consistent with the natural diphtheria toxin.

The diphtheria toxin mutant CRM197 of the present invention:

GADDVVDSSKSFVMENFSSYHGTKPGYVDSIQKGIQKPKSGTQG
NYDDDWKEFYSTDNKYDAAGYSVDNENPLSGKAGGVVKVTYPGLTK
VLALKVDNAETIKKELGLSLTEPLMEQVGTEEFIKRFGDGASRVVLSLP
FAEGSSSVEYINNWEQAKALSVELEINFETRGKRGQDAMYEYMAQAC
AGNRVRRSVGSSLSCINLDWDVIRDKTKTKIESLKEHGPIKNKMSESPN
KTVSEEKAKQYLEEFHQTALEHPELSELKTVTGTNPVFAGANYAAWA
VNVAQVIDSETADNLEKTTAALSILPGIGSVMGIADGAVHHNTEEIVA
QSIALSSLMVAQAIPLVGELVDIGFAAYNFVESIINLFQVVHNSYNRPA
YSPGHKTQPFLHDGYAVSWNTVEDSIIRTGFQGESGHDIKITAENTPLPI
AGVLLPTIPGKLDVNKSKTHISVNGRKIRMRCRAIDGDVTFCRPKSPVY
VGNGVHANLHVAFHRSSSEKIHSNEISSDSIGVLGYQKTVDHTKVNSK
LSLFFEIKS (SEQ ID NO: 2).

### The conjugate of recombinant human epidermal growth factor (hEGF) and diphtheria toxin mutant (hEGF-CRM197)

The present invention provides a conjugate of recombinant human epidermal growth factor (hEGF) and diphtheria toxin mutant, and the conjugate has a structure as shown in Formula (I):

hEGF-L-CRM197 (I)

wherein,
hEGF is the recombinant human epidermal growth factor (hEGF);
CRM197 is the diphtheria toxin mutant CRM197;
L is a linker connecting the recombinant human epidermal growth factor with the diphtheria toxin mutant CRM197, which is a covalent bond formed by glutaraldehyde crosslinking; and
"-" is a chemical bond.

Wherein, the recombinant human epidermal growth factor (hEGF) is an EGF molecule or a mutant thereof containing human epidermal growth factor (hEGF) biological activity, and the EGF biological activity refers to the ability of the EGF molecule to stimulate or induce signal transduction in cells containing EGFR receptors. In a preferred embodiment of the present invention, the recombinant human epidermal growth factor (hEGF) has an amino acid sequence as shown in SEQ ID NO: 3; the diphtheria toxin mutant CRM197 has an amino acid sequence as shown in SEQ ID NO: 2.

### Recombinant hEGF-CRM197 vaccine formulation

The present invention also provides a recombinant hEGF-CRM197 tumor vaccine formulation, which comprises: (a) a conjugate of recombinant human epidermal growth factor (hEGF) and diphtheria toxin mutant, (b) a basic buffer with a pH range of 7.0-8.5, wherein the conductivity of the buffer is not exceed 10mS/cm; and (c) a protein stabilizer; wherein the protein stabilizer comprises: a surfactant polysorbate 20 (Tween 20), and an optional monosaccharide or disaccharide.

In the preferred embodiment of the present invention, the basic buffer is a phosphate buffer, with a phosphate concentration of 1-40 mmol/L; The concentration of polysorbate 20 in the formulation is 0.001% to 0.1% (v/v); The optional monosaccharide or disaccharide includes (but is not limited to) sucrose, glucose, mannitol, maltose, and a combination thereof. In another preferred embodiment, the monosaccharide or disaccharide is sucrose, and the concentration of sucrose in the formulation is 0-4%.

The recombinant hEGF-CRM197 vaccine formulation of the present invention is a tumor therapeutic formulation. Therefore, the terms "recombinant hEGF-CRM197 vaccine formulation" and "recombinant hEGF-CRM197 tumor therapeutic vaccine formulation" can be used interchangeably, both referring to a liquid formulation prepared in the present invention containing the conjugate of recombinant human epidermal growth factor (hEGF) and diphtheria toxin mutant (hEGF-CRM197).

### The preparation method of recombinant hEGF-CRM197 vaccine formulation

The present invention also provides a method for preparing the recombinant hEGF-CRM197 vaccine formulation, which comprises the following steps:
(i) providing a purified recombinant human epidermal growth factor (hEGF) protein and a diphtheria toxin mutant CRM197 protein;
(ii) reacting the EGF protein and CRM197 protein with a crosslinking agent glutaraldehyde at room temperature for 2-3 hours, thereby obtaining the hEGF-CRM197 conjugate;
(iii) purifying the hEGF-CRM197 conjugate by using ultrafiltration method, thereby obtaining the purified hEGF-CRM197 conjugate;
(iv) exchanging the purified hEGF-CRM197 conjugate into a basic buffer supplemented with a protein stabilizer by ultrafiltration, thereby obtaining the recombinant hEGF-CRM197 vaccine formulation.

The present invention adopts a chemical crosslinking method to couple the recombinant human epidermal growth factor (hEGF) protein with the diphtheria toxin mutant CRM197 protein, thereby obtaining a novel tumor therapeutic vaccine. Coupling hEGF with CRM197 through glutaraldehyde can help improve the immunogenicity of hEGF and induce the production of anti-hEGF antibodies in human body. The optimal liquid formulation for preparing the tumor therapeutic vaccine into a formulation has been screened out through multiple experiments.

The recombinant hEGF-CRM197 vaccine formulation prepared using the above method has a low proportion of polymers and high molecular weight uniformity. After accelerated treatment at 25 °C ± 2 °C and 37 °C ± 2 °C for 6 months, there were no changes in its quality attributes such as appearance, visible foreign objects, molecular weight distribution, protein content, EGF antigen content, and biological activity, so it can be stored stably for a long time.

### Composition and application method

The present invention also provides a composition, which comprises (a) the tumor therapeutic vaccine formulation of the first aspect of the present invention; and (b) a pharmaceutically acceptable carrier.

The composition of the present invention includes a pharmaceutical composition or a vaccine composition.

The composition is prepared as a liquid formulation, including but not limited to an injection, suspension, or spray, etc.

The pharmaceutical composition of the present invention comprises (or contains) a therapeutically effective amount of hEGF-CRM197 of the present invention.

The term "therapeutically effective amount" used herein refers to the amount of a therapeutic agent used to treat, alleviate, or prevent a target disease or condition, or the amount that exhibits a detectable therapeutic or preventive effect. This effect can be detected by, for example, antigen levels. The therapeutic effect also includes a reduction in physiological symptoms. The precise effective dose for a certain object depends on its body size and health status, the nature and severity of the disease, and the choice of therapeutic agents and/or combinations of therapeutic agents to be administered. Therefore, it is useless to specify an accurate effective amount in advance. However, for a given situation, conventional experiments can be used to determine the effective amount.

For the purpose of the present invention, an effective dose for administration to an individual is approximately 0.001 milligrams per kilogram to 10 milligrams per kilogram, preferably approximately 0.003 milligrams per kilogram to 0.1 milligrams per kilogram of body weight.

The pharmaceutical composition may further contain a pharmaceutically acceptable carrier. The term "pharmaceutically acceptable carrier" refers to a carrier used for administering therapeutic agents (such as hEGF-CRM197 of the present invention). This term refers to some drug carriers that they themselves do not induce the production of antibodies that are harmful to individuals receiving the composition, and do not exhibit excessive toxicity after administration. Suitable carriers can be large and slowly metabolized macromolecules such as proteins, polysaccharides, polylactic acid, polyglycolic acid, etc. These carriers are well known to those of ordinary skill in the art. A thorough discussion on pharmaceutically acceptable carriers or excipients can be found in Remington's Pharmaceutical Sciences (Mack Pub. Co., N.J. 1991).

Pharmaceutically acceptable carriers in a composition may include liquids such as water, saline, glycerol, and ethanol. In addition, these carriers may further contain an auxiliary substance, such as a wetting agent or an emulsifier, a pH buffer substance, etc. Generally, the composition may be made into an injectable agent, for example, a liquid solution or suspension; it may also be made into a solid form suitable for being formulated into a solution or suspension or a liquid excipient before injection. Liposomes are also included in the definition of pharmaceutically acceptable carriers.

The vaccine composition of the present invention comprises immune antigens (including hEGF-CRM197 of the present invention), and they are usually combined with "pharmaceutically acceptable carriers", including any carrier that itself does not induce the production of antibodies that are harmful to the individual receiving the composition. Suitable carriers are usually large and slowly metabolized macromolecules, such as proteins, polysaccharides, polylactic acid, polyglycolic acid, amino acid polymers, amino acid copolymers, lipid aggregates (such as oil droplets or liposomes), etc. These carriers are well known to those of ordinary skill in the art. In addition, these carriers may function as immunostimulants ("adjuvants").

Preferred adjuvants for enhancing the efficacy of the immune composition include but are not limited to: (1) aluminum salts (alum), such as aluminum hydroxide, aluminum phosphate, aluminum sulfate, etc.; (2) Water-in-oil emulsion formula, for example: (a) Montanide ISA 51VG, (b) Montanide ISA 720VG; (C) MF59; (3) saponin adjuvants; (4) complete Freund adjuvant (CFA) and incomplete Freund adjuvant (IFA); (5) cytokines such as interleukins (such as IL-1, IL-2, IL-4, IL-5, IL-6, IL-7, IL-12, etc.), interferons (such as γ interferon), granulocyte macrophage colony stimulating factor (GM-CSF), etc.; (6) detoxified variants of bacterial ADP-ribosylated toxins (such as *Escherichia coli* heat-labile toxin LT); and (7) other substances used as immunostimulants to enhance the effectiveness of the composition.

Vaccine compositions, including immunogenic compositions (for example, they may comprise antigens, pharmaceutically acceptable carriers, and adjuvants), typically contain diluents such as water, saline, glycerol, ethanol, etc. In addition, auxiliary substances such as wetting agents or emulsifiers, pH buffering substances, etc., can exist in such carriers. More specifically, vaccines including immunogenic compositions contain immunologically effective amount of immunogenic compositions, and other required components mentioned above. "Immunologically effective amount" refers to an amount that is effective for treatment given to an individual in a single dose or part of a continuous dose. The dosage can be determined based on the health and physiological status of the treated individual, the type of treated individual (such as human), the ability of the individual's immune system to synthesize antibodies, the required level of protection, vaccine preparation, the evaluation of medical conditions by the treating physician, and other relevant factors. It is expected that the dosage will be within a relatively wide range and can be determined through routine experiments.

Usually, vaccine compositions or immunogenic compositions can be made into injectable formulations, such as liquid solutions or suspensions; It can also be made into solid forms suitable for adding solutions, suspensions, or liquid excipients before injection. The formulation may also be emulsified or encapsulated in liposomes to enhance the adjuvant effect.

Once the composition of the present invention is formulated, it can be directly given to the subject. The subject to be treated can be mammals, especially humans.

When used as a vaccine, the vaccine composition of the present invention can be directly applied to individuals using known methods, typically using the same administration routes as conventional vaccines. The routes for administering the pharmaceutical or vaccine composition of the present invention include (but are not limited to): subcutaneous, intradermal, intramuscular, or other parenteral administration routes. If necessary, the administration route can be combined or adjusted according to the condition of the disease. A vaccine composition can be administered in single or multiple doses, and may include administering booster doses to induce and/or maintain immunity. The vaccine composition should be administered in an "effective amount", that is, the amount of the vaccine composition in the chosen administration route is sufficient to trigger an immune response and effectively ameliorate disease symptoms.

Representative diseases include (but are not limited to) tumors (or cancers), especially solid tumors selected from the group consisting of: lung cancer, non-small cell lung cancer, colorectal cancer, breast cancer, liver cancer, gastric cancer, esophageal cancer, pancreatic cancer, melanoma, kidney cancer, prostate cancer, cervical cancer, ovarian cancer, nasopharyngeal cancer, oral cancer, osteosarcoma, cerebral glioma, bladder cancer, etc.

The amount of pharmaceutical compositions selected in each vaccine formulation is determined by an amount that can trigger an immune protective response without significant side effects. Usually, each dose of vaccines is sufficient to contain about 0.1mg-10mg, preferably 0.4mg-5mg. Standard research methods, including observing antibody titers and other reactions in subjects, can be used to determine the optimal dosage of a specific vaccine. The need for a booster dose can be determined by monitoring the immune level provided by the vaccine. After evaluating the antibody titer in the serum, it may be necessary to choose a booster dose of immunization. The administration of the pharmaceutical or vaccine composition can enhance the immune response in the present invention.

Furthermore, the vaccine of the present invention can be administered in combination with other immune modulators or in combination with other therapeutic agents.

Compared with conventional formulations, the main innovation of the present invention is to develop and provide a stable formulation containing human epidermal growth factor and diphtheria toxin mutant conjugate, which has the following main advantages:
(1) The formulation of the present invention contains an innovative component of the conjugate of human epidermal growth factor and diphtheria toxin mutant, which can break immune tolerance and induce the production of anti-human epidermal growth factor antibodies in the human body;
(2) The formulation of the present invention contains a low-salt and relatively alkaline basic buffer, which is significantly different from conventional formulations, and can produce a lower proportion of polymers in protein conjugate molecules, making the molecular weight distribution in the buffer more uniform;
(3) The present invention has explored through experiments the formula for preparing a recombinant hEGF-CRM197 tumor therapeutic vaccine formulation using polysorbate 20 and excluding polysorbate 80, thereby improving the stability of protein conjugate molecules in the formulation.
(4) The formulation production method of the present invention is simple, enabling the large-scale production and long-term stable preservation of the formulation.

The present invention is further explained below in conjunction with specific examples. It should be understood that these examples are only for illustrating the present invention and not intend to limit the scope of the present invention. The conditions of the experimental methods not specifically indicated in the following examples are usually in accordance with conventional conditions such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1: Preparation and detection of recombinant hEGF-CRM197 vaccine stock solution

The recombinant human epidermal growth factor, diphtheria toxin mutant CRM197, and crosslinking agent glutaraldehyde were added separately into a reaction vessel. After reaction at room temperature for 2-3 hours, free glutaraldehyde and unbound human epidermal growth factor were removed using ultrafiltration purification method to obtain the recombinant hEGF-CRM197 vaccine stock solution.

### (1) Preparation of recombinant hEGF-CRM197 conjugate

① Conjugation reaction: An appropriate amount of 25% glutaraldehyde was taken to prepare glutaraldehyde with a concentration of 0.5% in phosphate buffer (PBS: 20mM PB, 150mM NaCl, pH 7.0). Equal amounts of recombinant human epidermal growth factor and diphtheria toxin mutant CRM197 (with a molar ratio of approximately 10:1) were taken, respectively, and diluted to 2mg/ml in phosphate buffer. The recombinant human epidermal growth factor and diphtheria toxin mutant CRM197, which have been prepared into corresponding concentrations, were added to the reaction vessel, respectively. After thorough mixing, 0.5% glutaraldehyde was added dropwise until the final concentration of glutaraldehyde reached 0.1%. The reaction vessel was placed in a 25 °C constant temperature shaker, with a speed set to 130 rpm, and subjected to react for 2 hours.
② Ultrafiltration purification: After the reaction, a 50kDa ultrafiltration membrane was used for concentration first, then dilution was conducted using phosphate (PBS), and concentration was conducted again. Concentration was continued repeatedly, ensuring that the dilution ratio reached at least 2187 times (3 to the power of 7).
③ Basic buffer replacement: the ultrafiltration membrane was used continuously, and phosphate buffer (PB: 20mM PB, pH 8.0) was used for dilution and concentration, ensuring that the conductivity of the filtrate did not exceed 3mS/cm after the buffer exchange process. Finally, the ultrafiltration solution was concentrated to a concentration of at least 2mg/ml for the recombinant hEGF-CRM197 conjugate, ensuring compliance with the requirements for formulation preparation.

### (2) Protein crosslinking molar ratio detection of recombinant hEGF-CRM197 conjugate

Method: Liquid chromatography-tandem mass spectrometry (LC-MS/MS)

### ① Reductive alkylation

A sample of 50 µg after glycosidic bond cleavage was taken to adjust the pH to 7.5-8.0 with a 50 mmol/L ammonium bicarbonate solution. Then 100 mmol/L dithiothreitol (DTT) was added to achieve a final concentration of 10 mmol/L. The mixture was homogenized and incubated at 56 °C for 1 hour. Subsequently, 250 mmol/L of iodoacetamide (IAM) was added to achieve a final concentration of 25 mmol/L, and the mixture was subjected to a reaction at room temperature in the dark for 1 hour.

### ② Trypsin enzymatic digestion

50 µg of reduced alkylated sample was taken and added with Trypsin solution at a ratio of 1:50 (enzyme to protein). After thorough mixing, the solution was subjected to digestion at 37 °C for 12 hours. FA was then added to achieve a final concentration of 0.1% and terminate the reaction.

### ③ QE-HF data collection

An appropriate amount of enzymatic digestion solution was taken and centrifuged at 10000g for 5 minutes. The supernatant was collected for QE-HF analysis.

### ④ Data processing

All peptide segments and intensities thereof were extracted from the Mascot search results for each sample. The identical peptide segments among 6 batches of samples were selected to calculate the relative ratios of total rhEGF to CRM197 based on the number and intensity of detected peptide segments, respectively, and the ratios were then converted into molar ratios based on the molecular weights of rhEGF and CRM197. The results showed that the molar ratio of rhEGF/CRM197 in the recombinant hEGF-CRM197 conjugate was measured to be 6:1.

### (3) Antibody titer detection of mice immunized with recombinant hEGF-CRM197 conjugate

The recombinant hEGF-CRM197 conjugate stock solution was taken and the concentration of the recombinant hEGF-CRM197 vaccine was diluted to 0.2mg/ml. 0.5ml of the diluted vaccine was drawn into a 2ml syringe, and 0.5ml of the liquid adjuvant Montanide ISA 51 VG was drawn into another 2ml syringe. The two syringes were connected with a connector, and they were first pushed back and forth slowly for 15 cycles, and then as quickly as possible for 30 times to complete the emulsification. After emulsification, all emulsion was pushed into the syringe on one side. The emulsion was injected subcutaneously into a total of 8 mice at a volume of 0.1ml/mouse, and another injection was conducted with a weekly interval. Blood samples were collected from the orbital plexus of the mice at 1, 3, and 5 weeks after the second injection, and the anti-EGF antibody titers were measured subsequently.

Anti-EGF antibody titer detection method:
① Coating: The 96-well plates for ELISA detection were used, and the rhEGF was diluted to 0.5 µg/ml in Na₂CO₃-NaHCO₃ coating buffer with a pH of 9.6. Then, 100 µL of the diluted rhEGF protein was added to each well of the 96-well plates, and the plates were incubated overnight at 2-8 °C.
② Blocking: The next day, the coating solution was discarded and 150 µL of PBS blocking solution containing 0.05% (v/v) Tween-20 and 1% BSA was added to each well. The plates were incubated at 37 °C for 1 hour for blocking;
③ Washing: After discarding the blocking solution, each well was washed three times with 200 µL of PBS containing 0.05% (v/v) Tween-20, and the liquid in the wells was absorbed;
④ The collected mouse serum was diluted, and 100 µL of the diluted serum was taken and added to the leftmost well of the 96 well plates. Each mouse's serum was diluted in a row from left to right, with a total of 12 dilution gradients. The unimmunized mouse serum was used as a control. After adding the serum, the plates were incubated at 37 °C for 1 hour; After incubation, the incubation solution was discarded and the plates were washed repeatedly three times using the washing method described above;
⑤ The secondary antibody, HRP enzyme-labeled anti-mouse antibody, was diluted to 1:5000 using the blocking solution. 100 µL of HRP enzyme-labeled antibody was added to each well, and the plates were incubated at 37 °C for 1 hour; The incubation solution was then discarded and the plates were washed repeatedly 5 times using the washing method described above;
⑥ Color development: 100 µL TMB was added to each well and the plates were incubated at room temperature in the dark for 15 minutes;
⑦ Termination: 100 µL of 0.5mol/L sulfuric acid was added to each well to terminate the color development;
⑧ After termination, the optical density (OD) values at 450 nm (OD450) of each well were measured using a microplate spectrophotometer. The OD values were measured at 450 nm to determine the highest dilution ratio where serum sample OD value/physiological saline control group sample OD value was ≥ 2.1.

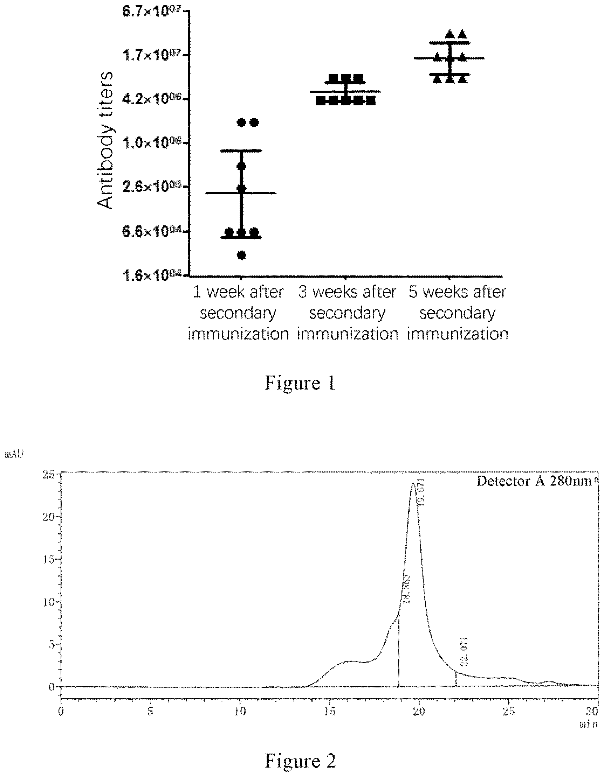

The test results are shown in Figure 1, indicating that the antibody titers exceeded 16,000 at 1, 3, and 5 weeks after secondary immunization, with geometric averages reaching 215,000, 5.31 million, and 15.02 million, respectively.

### Example 2: Screening of recombinant hEGF-CRM197 vaccine formulations

### (1) Basic buffer screening:

The prepared recombinant hEGF-CRM197 vaccine stock solution was taken and exchanged into the basic buffers shown in Table 1 below using the ultrafiltration exchange method, respectively. The basic buffers can significantly affect the molecular weight distribution of the recombinant hEGF-CRM197 vaccine stock solution. The screening goal of this study is to obtain a basic buffer with a low polymer ratio and high molecular weight uniformity. Therefore, SEC-HPLC analysis method was selected in this study to analyze the recombinant hEGF-CRM197 vaccine stock solution in different basic buffers using gel filtration analysis columns.

As shown in Table 1, the ratio of polymers reached the lowest at conditions of higher pH, lower salt concentration, and the absence of free amino acids, such as the buffer indicated by number 13.

**Table 1.**

| No. | Buffer | Polymer ratio |
|---|---|---|
| 1 | 20 mM PBS, pH 7.0 | 31.8% |
| 2 | 50 mM PBS, pH 7.6 | 33.5% |
| 3 | 100 mM PBS, pH 7.6 | 30.4% |
| 4 | 50 mM PBS, pH 8.0 | 28.0% |
| 5 | 50 mM PBS, 0.1 M Glycine, pH 7.0 | 31.5% |
| 6 | 50 mM PBS, 0.1 M Glycine, pH 7.6 | 28.6% |
| 7 | 1.15 g/L Na₂HPO₄, 8 g/L NaCl, 0.2 g/L KCl, 0.2 g/L KH₂PO₄ | 24.7% |
| 8 | 50 mM PBS, 0.1 M Argine, PH 7.6 | 32.3% |
| 9 | 25 mM Arg, 25 mM Glu, 150 mM NaCl, PH 7.6 | 31.6% |
| 10 | 20 mM PB, pH 7.6 | 26.0% |
| 11 | 1.28 g/L Na₂HPO₄, 8 g/L NaCl, 0.3 g/L KCl, 0.05 g/L KH₂PO₄, pH 8.0 | 28.1% |
| 12 | 1.28 g/L Na₂HPO₄, 0.3 g/L KCl, 0.05 g/L KH₂PO₄, pH 8.0 | 22.0% |
| 13 | 20 mM PB, pH 8.0 | 20.0% |

### (2) Protein stabilizer screening:

The recombinant hEGF-CRM197 vaccine stock solution was taken and formulated by adding corresponding amounts of high concentration protein stabilizer stock solutions as shown in Table 2, respectively. After sterilization and filtration, they were divided into 0.5ml/vial, with 20 vials in each group. An extreme accelerated stability test was conducted at 37 °C, and a preliminary screening was performed according to appearance and visible foreign objects after 2 weeks of accelerated treatment.

As shown in Table 2, the formulations containing polysorbate 80 (Tween 80) and polysorbate 20 were both qualified for visible foreign objects, and the addition of saccharides also improved stability to a certain extent.

**Table 2.**

| No. | Formulation | Vaccine concentration (mg/ml) | Appearance qualification rate (%) | Qualification rate of visible foreign objects (%) |
|---|---|---|---|---|
| a0 | 20mM PB, pH8.0 | 1.6 | 100 | 20 |
| a1 | 2% sucrose, 20mM PB, pH8.0 | 1.6 | 100 | 50 |
| a2 | 2% maltose, 20mM PB, pH8.0 | 1.6 | 100 | 30 |
| a3 | 1% sorbitol, 20mM PB, pH8.0 | 1.6 | 100 | 45 |
| a4 | 0.1% Tween80, 20mM PB, pH 8.0 | 1.6 | 100 | 100 |
| a5 | 4% sucrose, 20mM PB, pH8.0 | 1.6 | 100 | 45 |
| a6 | 10% sucrose, 20mM PB, pH8.0 | 1.6 | 100 | 30 |
| a7 | 0.1% Tween80, 2% sucrose, 20mM PB, pH8.0 | 1.6 | 100 | 100 |
| a8 | 4% sucrose, 20mM PB, pH7.0 | 1.6 | 100 | 25 |
| a9 | 10% glycerol, 20mM PB, pH8.0 | 1.6 | 100 | 10 |
| a10 | 10% glycerol, 20mM PB, 2% sucrose, pH8.0 | 1.6 | 100 | 15 |
| a11 | 10% glycerol, 2% sucrose, 0.1% Tween80, 20mM PB, pH8.0 | 1.6 | 100 | 90 |
| a12 | 0.001% Tween80, 2% sucrose, 20mM PB, pH8.0 | 1.6 | 100 | 100 |
| a13 | 0.1% Tween20, 1% sucrose, 20mM PB, pH8.0 | 1.6 | 100 | 100 |
| a14 | 0.1% Tween20, 4% sucrose, 20mM PB, pH8.0 | 1.6 | 100 | 100 |
| a15 | 0.1% Tween20, 2% sucrose, 5mM PB, pH8.0 | 1.6 | 100 | 100 |
| a16 | 0.05% Tween20, 20mM PB, pH8.0 | 1.6 | 100 | 100 |
| a17 | 0.02% Tween20, 2% sucrose, 5mM PB, pH8.0 | 1.6 | 100 | 100 |
| a18 | 0.01% Tween20, 2% sucrose, 5mM PB, pH8.0 | 1.6 | 100 | 100 |
| a19 | 0.005% Tween20, 2% sucrose, 5mM PB, pH8.0 | 1.6 | 100 | 100 |
| a20 | 0.1% Tween20, 2% sucrose, 20mM PB, pH8.0 | 1.6 | 100 | 100 |
| a21 | 0.01% Tween20, 2% sucrose, 20mM PB, pH8.0 | 1.6 | 100 | 100 |
| a22 | 0.005% Tween20, 2% sucrose, 20mM PB, pH8.0 | 1.6 | 100 | 100 |
| a23 | 0.001% Tween20, 2% sucrose, 20mM PB, pH8.0 | 1.6 | 100 | 100 |
| a24 | 0.1% Tween20, 2% sucrose, 20mM PB, pH8.0 | 3.2 | 100 | 100 |
| a25 | 0.01% Tween20, 2% sucrose, 20mM PB, pH8.0 | 0.8 | 100 | 100 |
| a26 | 0.005% Tween20, 2% sucrose, 20mM PB, pH8.0 | 0.4 | 100 | 100 |

The formulation formulas with 100% qualified appearance and visible foreign objects were further subjected to accelerated treatment at 37 °C for another month, and were then identified using methods such as visible foreign objects, protein content, molecular weight distribution (polymer ratio and principal component ratio), and *in vitro* relative potency.

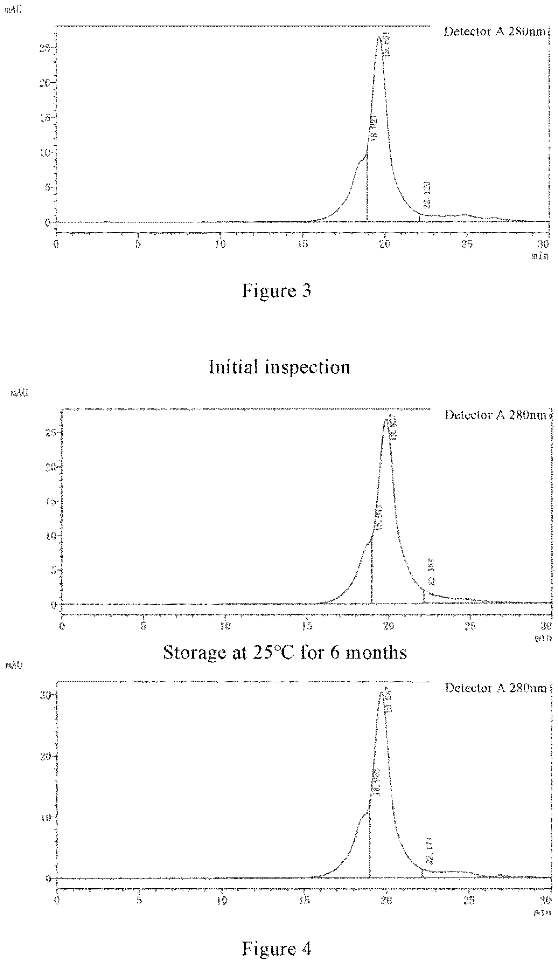

As shown in Table 3, it can be seen that all test indicators including the visible foreign objects, protein content, molecular weight distribution-principal component, and *in vitro* relative potency, met the stability requirements. In the accelerated stability test results of the formula containing polysorbate 80 (Tween 80), there was a significant increase in the polymer proportion of molecular weight distribution (as detailed in Table 3, as shown in Figure 2). In the accelerated stability test results of the formula containing polysorbate 20 (Tween 20), there was no significant change in the polymer proportion of molecular weight distribution (as detailed in Table 3, as shown in Figure 3). This result indicates that the formulation in a phosphate buffer containing polysorbate 20 is stable under extreme high temperature conditions.

**Table 3.**

| No. | Formulation | Qualificati on rate of visible foreign objects (%) | Proportion of protein content relative to initial inspection (%) | Poly mer ratio (%) | Principal compone nt ratio (%) | Proportion of *in vitro* relative potency relative to initial inspection (%) |
|---|---|---|---|---|---|---|
| a4 | 0.1%Tween8 0, 20mM PB, pH 8.0 | 100 | 95 | 29.1 | 92.3 | 89 |
| a7 | 0.1% Tween80, 2% sucrose, 20mM PB, pH8.0 | 100 | 101 | 30.0 | 93.5 | 110 |
| a12 | 0.001% Tween80, 2% sucrose, 20mM PB, pH8.0 | 100 | 102 | 25.6 | 92.6 | 106 |
| a13 | 0.1% Tween20, 1% sucrose, 20mM PB, pH8.0 | 100 | 97 | 23.3 | 94.1 | 95 |
| a14 | 0.1% Tween20, 4% sucrose, 20mM PB, pH8.0 | 100 | 96 | 21.5 | 92.8 | 88 |
| a15 | 0.1% Tween20, 2% sucrose, 5mM PB, pH8.0 | 100 | 96 | 20.9 | 93.5 | 102 |
| a16 | 0.05% Tween20, 20mM PB, pH8.0 | 100 | 98 | 20.7 | 92.8 | 104 |
| a17 | 0.02% Tween20, 2% sucrose, 5mM PB, pH8.0 | 100 | 101 | 21.1 | 94.2 | 94 |
| a18 | 0.01% Tween20, 2% sucrose, 5mM PB, pH8.0 | 100 | 105 | 20.7 | 92.1 | 101 |
| a19 | 0.005% Tween20, 2% sucrose, 5mM PB, pH8.0 | 100 | 97 | 20.8 | 93.3 | 97 |
| a20 | 0.1% Tween20, 2% sucrose, 20mM PB, pH8.0 | 100 | 102 | 21.1 | 93.5 | 93 |
| a21 | 0.01% Tween20, 2% sucrose, 20mM PB, pH8.0 | 100 | 97 | 20.5 | 92.9 | 109 |
| a22 | 0.005% Tween20, 2% sucrose, 20mM PB, pH8.0 | 100 | 95 | 21.3 | 93.6 | 105 |
| a23 | 0.001% Tween20, 2% sucrose, 20mM PB, pH8.0 | 100 | 98 | 20.9 | 92.9 | 99 |
| a24 | 0.1% Tween20, 2% sucrose, 20mM PB, pH8.0 | 100 | 99 | 21.5 | 93.5 | 95 |
| a25 | 0.01% Tween20, 2% sucrose, 20mM PB, pH8.0 | 100 | 96 | 20.9 | 93.2 | 106 |
| a26 | 0.005% Tween20, 2% sucrose, 20mM PB, pH8.0 | 100 | 95 | 20.8 | 94.1 | 93 |

### Example 3: Preparation, activity identification, and stability study of the recombinant hEGF-CRM197 tumor therapeutic vaccine formulation (0.01% Tween20, 2% sucrose, 20mM PB, pH 8.0, 1.6mg/ml)

### 3.1 Preparation of formulation product

The content of the required stock solution and various excipients for the preparation of the recombinant hEGF-CRM197 tumor therapeutic vaccine was calculated based on the preparation amount and concentration. The recombinant hEGF-CRM197 tumor therapeutic vaccine stock solution, Tween 20 and sucrose stock solution were added to the preparation container, respectively, and finally the basic buffer (20mM PB) was added to make up to the volume.

After sterilization and filtration using a 0.2 µm pore size sterilization filter, the formulation product was divided into penicillin bottles under sterile conditions.

### 3.2 Detection of formulation product

### (1) Appearance and visible foreign objects

The inspector adjusted the position so that the test sample was located at a clear visual distance from the eyes, with a distance of 25 cm from the test sample to the human eyes. The container label was removed and the outer wall of the container was wiped clean. The inspector held the neck of the container in hand, gently rotated and inverted the container to suspend any visible foreign objects present in the solution, taking care not to create bubbles in the solution. Visual inspection was performed under both black and white backgrounds, repeating 3 times for a total time limit of 20 seconds.

No obvious foreign objects shall be detected in the test sample. If no microscopic visible foreign objects were detected, it was deemed to comply with the regulations.

Explanation of microscopic visible foreign objects: Firstly, white dots refer to white objects that cannot distinguish flat or sharp edges; Secondly, small protein floccules or protein particles refer to translucent flocculent precipitates or protein particles smaller than about 1 mm; Thirdly, a small amount of floccules or protein particles refer to protein floccules or protein particles that are difficult to count within a specified inspection time; Fourthly, trace sediments refer to tiny precipitates in the test sample that, after standing, float up as smoke-like precipitates when gently rotated and dissipate with gentle shaking; Fifthly, non-dispersible precipitates refer to a small amount of precipitates that appear in the protein solution after prolonged standing and do not disperse or disappear after gentle shaking.

### (2) Protein content

Standard protein solutions of 0.2 mg/ml, 0.4 mg/ml, 0.6 mg/ml, 0.8 mg/ml, and 1.0 mg/ml were precisely pipetted into 96-well plates at 6 µl, respectively. Two replicate wells were set for each standard, which means each standard was tested three times; The test samples were also precisely pipetted into 96-well plates at 6 µl, respectively. Two replicate wells were set for each test sample, which means each test sample was tested three times; 30 µl of reagent A was added to each well in sequence, shaken well; 180 µl of reagent B was added to each well in sequence, shaken well, and let the plate stand at room temperature for 10 minutes. The OD750 absorbance values were measured at a wavelength of 750 nm.

A standard curve was drawn based on the OD750 absorbance values of the standard protein solutions, obtaining a linear regression equation, and the OD750 absorbance value of the test sample was substituted into the linear regression equation to obtain the protein content of the test sample.

### (3) Purity (SDS-PAGE)

The test sample was taken and mixed with the loading buffer at a ratio of 3:1, and then subjected to a boiling water bath for 5 minutes. The sample was analyzed using a gel with a 12.5% concentration of acrylamide and constant voltage electrophoresis at 200 V was conducted for 45 minutes.

After electrophoresis, the protein bands in the gel could be colored by Coomassie brilliant blue staining and scanned for imaging. The proportion of recombinant hEGF-CRM197 vaccine bands with a molecular weight not less than that of the recombinant CRM197 bands to all components was calculated as the principal component ratio.

### (4) Molecular weight distribution (SEC-HPLC)

### ① Pre-treatment of test samples

The test sample was taken and centrifuged at 10000rpm for 10 minutes before collecting the supernatant.

### ② Sample analysis

Program running time: 40 minutes
Data recording time: 30 minutes
Flow rate: 0.5 ml/min

The formulation product was taken for loading, the analysis method was set according to the above parameters for analysis, and the proportion of polymer area and the proportion of principal component area were calculated for the analysis results by using software.

### (5) In vitro relative potency

① The reference sample and formulation product were diluted with Na₂CO₃-NaHCO₃ buffer to 50, 25, 12.5, 6.25, and 3.13 ng/ml, respectively. Each batch of vaccines with 5 gradients of dilution were coated on ELISA plates, with 2 replicate wells for each dilution and 100 µl for each well, and the plates were then incubated overnight at 2-8 °C. Then the plates were washed with PBST and patted dry. The plates were blocked with 200 µl of blocking solution and incubated at 37 °C for 2 hours.
② The pre-coated ELISA plates were taken and added with 1 µg/ml of S-172-4 hybridoma monoclonal antibody, and then incubated at 37 °C for 1 hour. After washing the plates, 1:10000 diluted goat anti-mouse IgG-HRP was added, with 100 µl per well, and the plates were incubated at 37 °C for 1 hour.
③ After washing the plates, 100 µl/well TMB was added and the plates were incubated in the dark for 15 minutes. Then, 50 µl/well termination solution was added and the OD values were read at 450 nm.
④ Each batch of samples was tested three times.
(6) Biological activity (same method as Example 1, but only detecting mouse serum collected 1 week after secondary immunization)

The formulation product was taken and the concentration of the recombinant hEGF-CRM197 vaccine was diluted to 0.2mg/ml. 0.5ml of the diluted vaccine was drawn into a 2ml syringe, and 0.5ml of the liquid adjuvant Montanide ISA 51 VG was drawn into another 2ml syringe. The two syringes were connected with a connector, and they were first pushed back and forth slowly for 15 cycles, and then as quickly as possible for 30 times to complete the emulsification. After emulsification, all emulsion was pushed into the syringe on one side. The emulsion was injected subcutaneously into a total of 8 mice at a volume of 0.1ml/mouse, and another injection was conducted with a weekly interval. Blood samples were collected from the orbital plexus of the mice at 1 week after the second injection, and the anti-EGF antibody titers were measured subsequently.

Anti-EGF antibody titer detection method:
① Coating: The 96-well plates for ELISA detection were used, and the rhEGF was diluted to 0.5 µg/ml in Na₂CO₃-NaHCO₃ coating buffer with a pH of 9.6. Then, 100 µL of the diluted rhEGF protein was added to each well of the 96-well plates, and the plates were incubated overnight at 2-8 °C.
② Blocking: The next day, the coating solution was discarded and 150 µL of PBS blocking solution containing 0.05% (v/v) Tween-20 and 1% BSA was added to each well. The plates were incubated at 37 °C for 1 hour for blocking;
③ Washing: After discarding the blocking solution, each well was washed three times with 200 µL of PBS containing 0.05% (v/v) Tween-20, and the liquid in the wells was absorbed;
④ The collected mouse serum was diluted, and 100 µL of the diluted serum was taken and added to the leftmost well of the 96 well plates. Each mouse's serum was diluted in a row from left to right, with a total of 12 dilution gradients. The unimmunized mouse serum was used as a control. After adding the serum, the plates were incubated at 37 °C for 1 hour; After incubation, the incubation solution was discarded and the plates were washed repeatedly three times using the washing method described above;
⑤ The secondary antibody, HRP enzyme-labeled anti-mouse antibody, was diluted to 1:5000 using the blocking solution. 100 µL of HRP enzyme-labeled antibody was added to each well, and the plates were incubated at 37 °C for 1 hour; The incubation solution was then discarded and the plates were washed repeatedly 5 times using the washing method described above;
⑥ Color development: 100 µL TMB was added to each well and the plates were incubated at room temperature in the dark for 15 minutes;
⑦ Termination: 100 µL of 0.5mol/L sulfuric acid was added to each well to terminate the color development;
⑧ After termination, the optical density (OD) values at 450 nm (OD450) of each well were measured using a microplate spectrophotometer. The OD values were measured at 450 nm to determine the highest dilution ratio where serum sample OD value/physiological saline control group sample OD value was ≥ 2.1.

### 3.3 Researches on the stability of formulation product

The formulation product was selected, and after completing the initial inspection using the method described in 3.2, a 25 °C accelerated stability study was conducted according to the scheme described in Table 4 below. The inspection methods for all test items are described in 3.2.

**Table 4.**

| **Test items** | **1 mon th** | **2 mon ths** | **3 mon ths** | **6 mon ths** |
|---|---|---|---|---|
| Appearance | √ | √ | √ | √ |
| Visible foreign objects | √ | √ | √ | √ |
| Protein content | √ | √ | √ | √ |
| Purity (SDS-PAGE) | × | × | | |
| Molecular weight distribution (SEC-HPLC) | √ | √ | √ | √ |
| Biological activity determination | × | × | × | |
| *In vitro* relative potency | × | × | | |

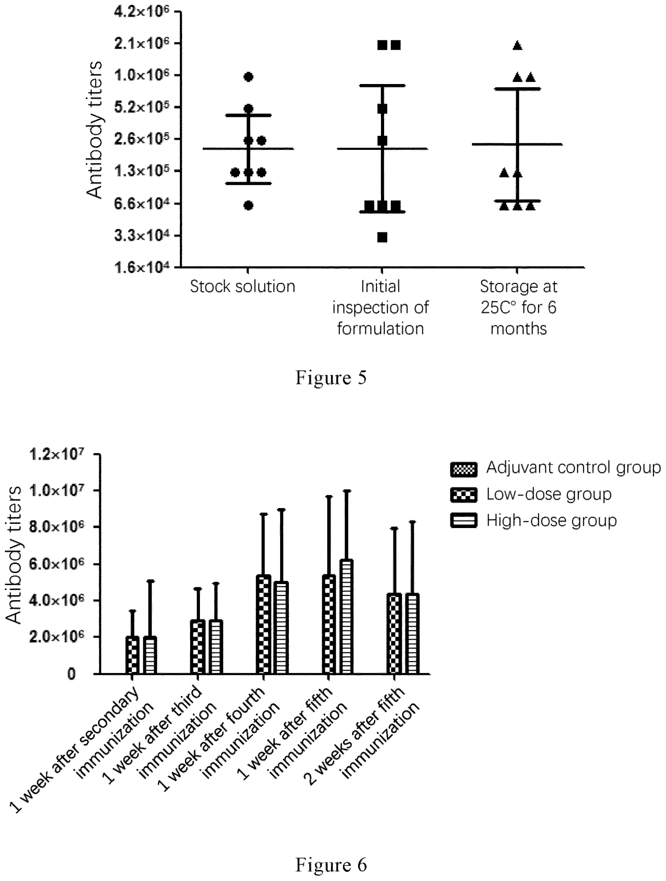

After completing 6 months of stability study, the research results were summarized, as shown in Table 5. In the 6-month accelerated stability study of the formulation product, key quality parameters such as appearance, visible foreign objects, protein content, purity (SDS-PAGE), molecular weight distribution (SEC-HPLC) (Figure 4), *in vitro* relative potency, and *in vivo* biological activity (Figure 5) were all within the controllable quality range.

**Table 5.**

| **Test items** | **Initial inspection** | **1 month** | **2 months** | **3 months** | **6 months** |
|---|---|---|---|---|---|
| Appearance | 100% qualified | 100% qualified | 100% qualified | 100% qualified | 100% qualified |
| Visible foreign objects | 100% qualified | 100% qualified | 100% qualified | 100% qualified | 100% qualified |
| Protein content (mg/ml) | 1.60 | 1.62 | 1.57 | 1.61 | 1.64 |
| Purity (SDS-PAGE) | 97.2% | × | × | 99.1% | 98.7% |
| Molecular weight distribution (SEC-HPLC)-prin cipal component ratio (Figure 3) | 93.3% | 91.5% | 91.5% | 93.0% | 92.8% |
| Molecular weight distribution (SEC-HPLC)-poly mer ratio (Figure 3) | 19.5% | 20.5% | 23.3% | 22.9% | 23.5% |
| Biological activity determination (geometric mean of antibody titers) (Figure 4) | 2.2×10⁵ | × | × | × | 2.3×10⁵ |
| *In vitro* relative potency | 0.92 | × | × | 0.88 | 1.04 |

### Example 4 Biological activity detection of recombinant hEGF-CRM197 tumor therapeutic vaccine formulation in cynomolgus monkey

Due to the high 98% homology of EGF sequences between humans and cynomolgus monkeys, studying the biological activity of recombinant hEGF-CRM197 tumor therapeutic vaccine formulation using cynomolgus monkey can preliminarily evaluate the biological activity of recombinant hEGF-CRM197 tumor therapeutic vaccine formulation in humans.

Preparation of test sample: The recombinant EGF-CRM197 vaccine was mixed with Montanide ISA 51 VG adjuvant in equal volume and then emulsified. The specific emulsification process is as follows:
Two 2ml syringes were taken and labeled as No.1 and No.2 syringes, respectively. Equal volumes of Montanide ISA 51 VG adjuvant and recombinant EGF-CRM197 vaccine were drawn into syringes No.1 and No.2, respectively, and the two syringes were connected using a double female Luer connector. The entire emulsification process was divided into two steps: "pre emulsification" and "rapid emulsification". The process of pushing all the liquid from syringe No.1 to syringe No.2, and then back to syringe No.1, was defined as a cycle. The "pre emulsification" process required 20 cycles, with the syringe slowly pushed back and forth for an average of about 8 seconds per cycle. It took at least 2 minutes to complete the "pre emulsification" process. After the "pre emulsification" was completed, it entered the "rapid emulsification" process. The "rapid emulsification" process consisted of 40 cycles, and the syringe needed to be pushed back and forth as quickly as possible. The whole emulsification process took 60 cycles and took about 3 minutes. The final water-in-oil emulsion was milky white and viscous. After emulsification, all the water-in-oil emulsion was pulled into a 1.5ml centrifuge tube and it was placed in an ice box for subsequent use. The test sample was mixed thoroughly with an equal volume of adjuvant until fully emulsified.

**Experimental animals: 30 cynomolgus monkeys, 10 in each group, aged 3-5 years old, weighing 2-4kg; They were the adjuvant control group, low-dose group, and high-dose group, respectively.**
Administration route and method: subcutaneous injection into the neck and back;
Frequency and duration of administration: once a week for a total of 5 times;
Administration time: generally from 08:30 to 12:00 am;
Dosage concentration: 0.5 mg/ml;
Dosage volume: 0.8 ml/animal in the low-dose group, and 1.6 ml/animal in all other groups.

Blood was collected weekly for 1-5 weeks after secondary administration.

The antibody titer determination method is the same as the anti-EGF antibody titer detection method in Example 1.

The antibody titer determination results are shown in Figure 6. The results showed that the recombinant hEGF-CRM197 tumor therapeutic vaccine formulation could induce cynomolgus monkeys to produce up to 5 million anti-EGF antibody titers, indicating that the recombinant hEGF-CRM197 tumor therapeutic vaccine formulation can break the immune tolerance of cynomolgus monkeys and induce them to produce high titer antibodies, thereby verifying the *in vivo* biological activity of the recombinant hEGF-CRM197 tumor therapeutic vaccine formulation.

The EGF content in the cynomolgus monkey body was further detected, and the detection method for EGF content in serum is as follows:
Experimental preparation: Before use, all reagents were placed at room temperature and the Human EGF standard was dissolved in 5.0 ml of Calibrator Diluent RD6-1 to obtain a final concentration of 250 pg/ml. The Human EGF standard was serially diluted to 125 pg/ml, 62.5 pg/ml, 31.2 pg/ml, 15.6 pg/ml, 7.8 pg/ml, and 3.9 pg/ml using Calibrator Diluent RD6-1, and Calibrator Diluent RD6-1 was used as a standard at 0 pg/ml for plotting standard curves.

Sample addition, warm incubation: 50 µL/well Assay Diluent RD1-21 was first added, then 200 µL/well standard, control, and sample solutions were added, respectively. The edge of the plates was gently tapped for 1 minute to mix the solution evenly. The plates were sealed with a sealing film and incubated at room temperature for 2 hours.

Washing: The sealing film was carefully removed, and the liquid was discarded. The plates were shaken dry, then added with 400 µL of washing buffer to each well, and the washing buffer was discarded. This process was repeated 3 times, then the plates were patted dry.

Enzyme addition: 200 µl of EGF Conjugate was added to each well, and the plates were sealed with a sealing film, and then incubated at room temperature for 2 hours.

Washing was conducted according to the above washing steps.

Color development: 200 µl of substrate solution was added to each well and the plates were incubated at room temperature in the dark for 20 minutes.

Termination: 50 µl of Stop Solution was added to each well, then the color was changed from blue to yellow. If the color in the well was green or uneven, the plates were gently shaken to evenly mix the solution.

Reading: The OD values were read at 450 nm.

The average value of the blank control was subtracted from the average reading of each standard, control, and sample's replicate wells. Standard solutions (250 pg/ml, 125 pg/ml, 62.5 pg/ml, 31.2 pg/ml, 15.6 pg/ml, 7.8 pg/ml, 3.9 pg/ml) could be used to create standard curves, and data analysis could use computer software to draw four-parameter logistic curves. Alternatively, a standard curve could be drawn based on the average absorbance value of each standard sample, with Y-axis annotation of absorbance value, and X-axis annotation of the concentration. Then the concentration of EGF in the sample was calculated based on its absorbance value. (During operation, each concentration of standard solution should be titrated repeatedly into two small wells to calculate the average spectrophotometry value, and the average absorbance value of the sample should also be calculated). The final concentration is obtained by multiplying the actual measured concentration by the dilution factor.

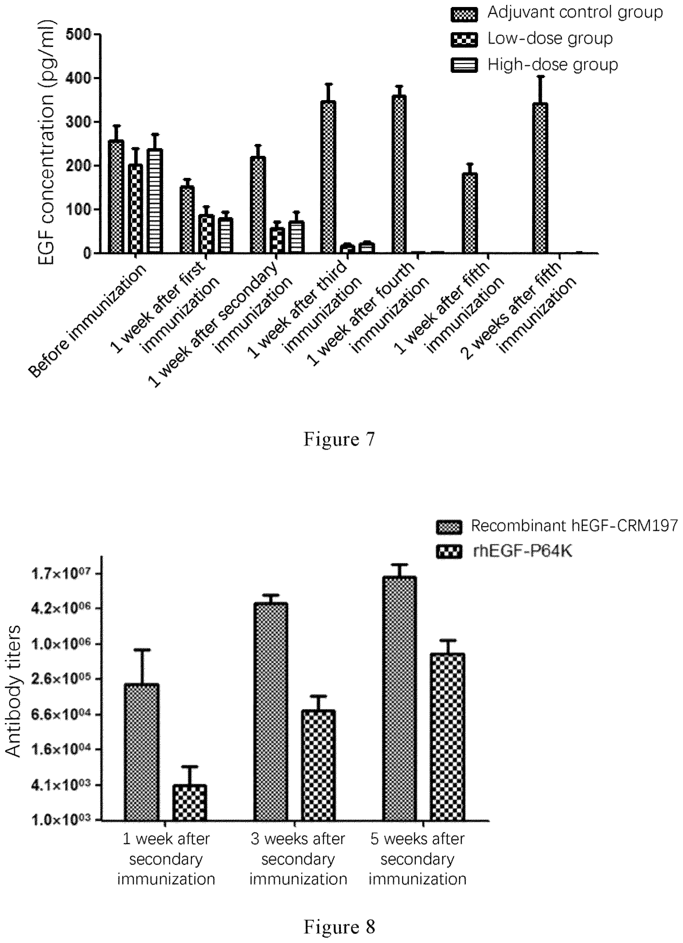

The detection results are shown in Figure 7. The results showed that the EGF content in the serum of cynomolgus monkeys in the vaccine group (high-dose group and low-dose group) significantly decreased, while there was no significant change in the EGF content in the adjuvant control group.

### Comparative Example 1: Comparison of biological activities between rhEGF-P64k conjugate (CIMAvax EGF) and recombinant hEGF-CRM197

According to the antibody titer detection method of mice immunized with the recombinant hEGF-CRM197 conjugate in Example 1, the rhEGF-P64k conjugate (CIMAvax-EGF) was prepared and used for animal immunization referring to the preparation and animal immunization methods of the recombinant hEGF-CRM197 vaccine. Serum was collected at 1 week, 3 weeks, and 5 weeks after secondary immunization using the same method, and antibody titers were measured using the same method. As shown in Figure 8, the antibody titers of the recombinant hEGF-CRM197 conjugate were significantly higher than those of the rhEGF-P64k conjugate by at least 10 times at 1 week, 3 weeks, and 5 weeks after secondary immunization, respectively.

Further comparison of the *in vitro* blocking ability of EGFR and EGF binding by anti-EGF antibodies induced by the CIMAvax EGF conjugate and the recombinant hEGF-CRM197 conjugate was conducted:
1 µl of FITC-EGF was incubated with serum of BALB/c mice immunized with CIMAvax EGF/recombinant hEGF-CRM197 tumor therapeutic vaccine collected at 3 weeks after the secondary immunization, or serum of BALB/c mice injected with normal saline collected at 3 weeks after the secondary immunization, for 15 minutes at room temperature in the dark, with volume ratios of 1:25, 1:75, and 1:100, respectively.

NCI-H446 cells in logarithmic growth phase were digested with trypsin and evenly divided into 11 tubes, with a number of approximately 10⁴ cells in each 1.5 ml centrifuge tube. The cells were centrifuged at 5000 rpm for 3 minutes, and the supernatant was discarded.

The incubated mixture of FITC-EGF and serum was mixed with NCI-H446 cells, respectively, and supplemented with normal saline to a total volume of 110 µl per tube, and then incubated at 4 °C in the dark for 30 minutes. Samples 1-9 were labeled, respectively, wherein samples 1-3 were labeled as normal saline group, samples 4-6 were labeled as EGF-P64K group, and samples 7-9 were labeled as EGF-CRM197 group. Only 110 µl of normal saline was added to sample 10 for flow cytometer zeroing, and 1 µl of FITC-EGF was added to sample 11, and supplemented with normal saline to a total volume of 110 µl per tube as a negative control.

1 ml of normal saline was added to the 11 samples respectively, centrifuged at 5000 rpm for 3 minutes to wash the cells, and the supernatant was discarded.

200 µl of normal saline was added to the 11 samples respectively. FACS was used to detect the positive proportion of NCI-H446 cells labeled with FITC-EGF in each sample.

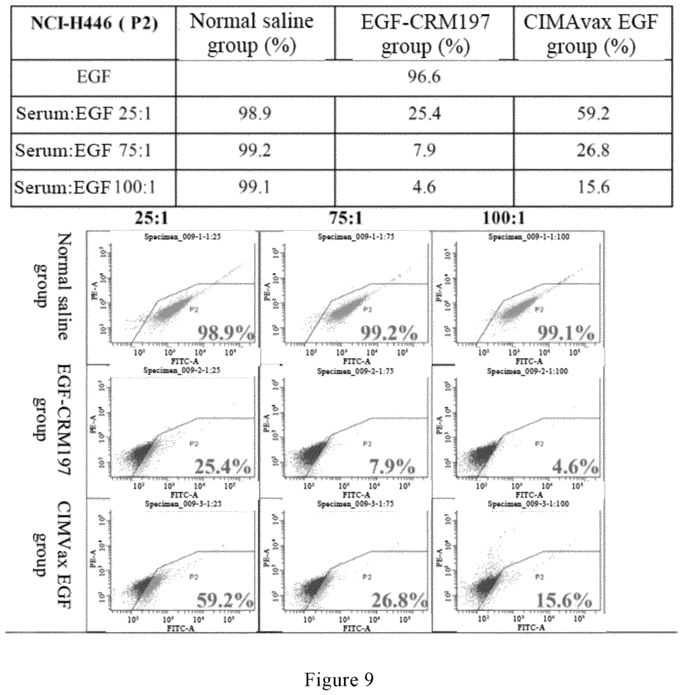

The results are shown in Figure 9. The results showed that after mixing and incubating the serum collected at 3 weeks after the secondary immunization of the recombinant EGF-CRM197/CIMAvax EGF vaccine group with FITC-EGF in volume ratios of 25:1, 75:1, and 100:1, the positive rates of NCI-H446 cells labeled with FITC-EGF were 25.4% vs. 59.2%, 7.9% vs. 26.8%, and 4.6% vs. 15.6%, respectively. The experimental results indicate that after immunization with the recombinant EGF-CRM197/CIMAvax EGF vaccine in mice, the induced antiserum can effectively block the binding of EGF and EGFR *in vitro.* Among them, the recombinant EGF-CRM197 vaccine group requires less serum to inhibit the same amount of EGF, indicating that the induced antibodies in the recombinant EGF-CRM197 vaccine group have a better binding ability. Compared to an inhibition rate of around 25%, the serum amount of the CIMAvax EGF group is three times higher than that of the recombinant EGF-CRM197 group, and it is roughly judged that the neutralizing antibodies induced by the recombinant EGF-CRM197 vaccine group are three times higher than those of the CIMAvax EGF group.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A recombinant hEGF-CRM197 tumor therapeutic vaccine formulation, which comprises:
(a) a recombinant human epidermal growth factor (hEGF)-diphtheria toxin mutant CRM197 conjugate, wherein the content of the conjugate is 0.4-3.2 mg/ml, and the conjugate is obtained by coupling a recombinant human epidermal growth factor (hEGF) and a diphtheria toxin mutant CRM197 with a chemical crosslinking agent glutaraldehyde, and the crosslinking molar ratio of the recombinant human epidermal growth factor (hEGF)/diphtheria toxin mutant CRM197 in the conjugate is 6:1;
(b) a phosphate basic buffer with a pH range of 7.5-8.5, wherein the conductivity of the buffer is not higher than 3mS/cm, and the phosphate concentration in the buffer is 5-20mmol/L; and
(c) a protein stabilizer;
wherein, the protein stabilizer comprises: 0.005%-0.02% surfactant polysorbate 20 and 0%-4% monosaccharide or disaccharide, calculated by the total volume of the formulation.

2. The formulation of claim 1, wherein the conjugate has a structure as shown in Formula (I):
hEGF-L-CRM197 (I)
wherein,
hEGF is the recombinant human epidermal growth factor (hEGF);
CRM197 is the diphtheria toxin mutant CRM197;
L is a linker connecting the recombinant human epidermal growth factor with the diphtheria toxin mutant CRM197, which is a covalent bond formed by glutaraldehyde crosslinking; and
"-" is a chemical bond.

3. The formulation of claim 1, wherein the recombinant human epidermal growth factor (hEGF) is an EGF molecule or a mutant thereof containing human epidermal growth factor (hEGF) biological activity, and the EGF biological activity refers to the ability of the EGF molecule to stimulate or induce signal transduction in cells containing EGFR receptors.

4. The formulation of claim 1, wherein an amino acid sequence of the recombinant human epidermal growth factor (hEGF) is the amino acid sequence as shown in SEQ ID NO: 3.

5. The formulation of claim 1, wherein an amino acid sequence of the diphtheria toxin mutant CRM197 is the amino acid sequence as shown in SEQ ID NO: 2.

6. The formulation of claim 1, wherein the monosaccharide or disaccharide is selected from the group consisting of: sucrose, glucose, mannitol, maltose, and a combination thereof.

7. The formulation of claim 1, wherein the formulation is an injection.

8. A method for preparing the recombinant hEGF-CRM197 tumor therapeutic vaccine formulation of claim 1, which comprises the steps of:
(i) providing a purified recombinant human epidermal growth factor (hEGF) protein and a diphtheria toxin mutant CRM197 protein;
(ii) reacting the hEGF protein and CRM197 protein with a chemical crosslinking agent glutaraldehyde, thereby obtaining the hEGF-CRM197 conjugate;
(iii) purifying the hEGF-CRM197 conjugate, thereby obtaining the purified hEGF-CRM197 conjugate;
(iv) exchanging the purified hEGF-CRM197 conjugate into a basic buffer supplemented with a protein stabilizer, thereby obtaining the recombinant hEGF-CRM197 tumor therapeutic vaccine formulation.

9. A composition comprising (a) the tumor therapeutic vaccine formulation of claim 1; and (b) a pharmaceutically acceptable carrier.

10. Use of the tumor therapeutic vaccine formulation of claim 1 in the manufacture of a medicament for treating and/or preventing a tumor or cancer.
